# EUROPEAN PATENT SPECIFICATION

(11) **EP 3 052 460 B1**
(45) Date of publication and mention of the grant of the patent: **04.12.2024**
(21) Application number: 14777310.5
(22) Date of filing: 29.09.2014
(51) Int. Cl.: C07C 1/00, B01J 37/34, B01J 21/04, B01J 23/44, B01J 23/63, B01J 37/02

(54) **PD ON BOEHMITE CATALYTIC SYSTEM FOR SELECTIVE HYDROGENATION OF TRIPLE BONDS**
NEUES KATALYTISCHES SYSTEM FÜR SELEKTIVE HYDRIERUNGEN
NOUVEAU SYSTÈME CATALYTIQUE POUR DES HYDROGÉNATIONS SÉLECTIVES

(30) Priority: 30.09.2013 EP 13186731
(43) Date of publication of application: 10.08.2016
(73) Proprietor: DSM IP Assets B.V., 6221 BE Maastricht (NL)
(72) Inventor: CRAVOTTO, Giancarlo, CH-4303 Kaiseraugst (CH); BONRATH, Werner, CH-4303 Kaiseraugst (CH); MEDLOCK, Jonathan Alan, CH-4303 Kaiseraugst (CH); BORETTO, Emily, CH-4303 Kaiseraugst (CH); WU, Zihilin, CH-4303 Kaiseraugst (CH)
(74) Representative: dsm-firmenich IP
(86) International application number: PCT/EP2014/070765
(87) International publication number: WO 2015/044411

(56) References cited:
- WO-A1-97/26989
- US-B1- 6 350 717
- MIN SERK KWON ET AL: "Palladium Nanoparticles Entrapped in Aluminum Hydroxide: Dual Catalyst for Alkene Hydrogenation and Aerobic Alcohol Oxidation", ORGANIC LETTERS, AMERICAN CHEMICAL SOCIETY, US, vol. 7, no. 6, 17 March 2005 (2005-03-17), pages 1077 - 1079, XP002577877, ISSN: 1523-7060, [retrieved on 20050225], DOI: 10.1021/OL047381W
- GARRY GLASPELL ET AL: "Microwave Synthesis of Supported Au and Pd Nanoparticle Catalysts for CO Oxidation", THE JOURNAL OF PHYSICAL CHEMISTRY B, vol. 109, no. 37, 1 September 2005 (2005-09-01), pages 17350 - 17355, XP055103596, ISSN: 1520-6106, DOI: 10.1021/jp0526849
- KENJI OKITSU ET AL: "Sonochemical Preparation and Catalytic Behavior of Highly Dispersed Palladium Nanoparticles on Alumina", CHEMISTRY OF MATERIALS, vol. 12, no. 10, 1 October 2000 (2000-10-01), pages 3006 - 3011, XP055103591, ISSN: 0897-4756, DOI: 10.1021/cm0001915
- ANONYMOUS, 1 January 2007 (2007-01-01), XP055166568, Retrieved from the Internet <URL:http://www.sigmaaldrich.com/technical-documents/articles/chemfiles/nanoparticulate-rh.printerview.html> [retrieved on 20150202]

## Description

The present invention relates to selective hydrogenations of C-C triple bonds to C-C double bonds.

Selective hydrogenations (reductions) are important reactions in the field of organic chemistry. The goal of such hydrogenations is getting compounds with C-C double bonds (and not further reducing them to single bonds).

Selective hydrogenations are known in the prior art. Usually there are carried out by using specific kind of catalytic systems. The terms catalytic systems and catalysts are used as synonyms in the context of this patent application.

The most important and most widely used types of catalysts are the Lindlar catalysts. A Lindlar catalyst is a heterogeneous catalyst that consists of palladium deposited on calcium carbonate or barium sulfate and treated with various forms of lead (Pb).

Because of the importance of selective hydrogenations there is always a need to provide improved catalytic systems and/or improved selective hydrogenation processes, which are hydrogenations (reductions) of C-C triple bonds to C-C double bonds. The C-C double bonds are not further hydrogenated to C-C single bonds. US 6 350 717 B1 discloses the selective hydrogenation of alkynes with Pd on alumina oxide.

Surprisingly it was found that a catalytic system wherein Pd-nanoparticles are deposited on a specific metal oxide carrier, excellent yields, selectivity and conversions can be achieved. Furthermore this catalytic system does not contain any lead at all. This means the catalytic system is essentially free from lead (Pb). This means that it can contain Pb in traces (but no Pb is added intentionally).

AIO(OH) is also known as Boehmite.

As stated above the catalytic system used in the process according to the present invention does not contain any lead at all. This is in contrast to the typical Lindlar catalyst wherein lead is added deliberately (as so called "poison") to obtain the desired results.

In the context of the present invention the terms "catalyst(s)" and "catalytic system(s)" are used synonymously.

Pd-nanoparticles are on the metal oxide carrier. Preferably, up to 8 weight-% (wt-%), based on the total weight of the catalytic system, of Pd is on the carrier material. Usually at least 0.01 wt-%, based on the total weight of the catalytic system, of Pd is on the carrier material. More preferably 0.01 - 5 wt-%, based on the total weight of the catalytic system, of Pd. Most preferably 0.1 - 3 wt-%, based on the total weight of the catalytic system, of Pd.

In the following these three catalyst preparation methods are discussed in further details.

### (1) Ultrasound-assisted reduction-by-solvent of Pd(II):

A Pd(ll) salt (or a mixture of Pd(ll) salts) are suspended/dispersed in a suitable solvent and then sonicated for a short period of time (usually at elevated reaction temperature) and afterwards this suspension/dispersion (or parts of it) is added to an aqueous suspension of carrier material (AIO(OH)). This mixture is stirred and afterwards the solid is filtered, washed and dried to obtain the catalytic system.

The sonication (treatment with ultrasound) can be carried by commonly known Ultrasound devices.

The frequency used in such process is usually more than 16kHz. The frequency used in such process is usually less than 200 kHz.

A preferred range of frequency is 16kHz to 150 kHz.

It is clear that during the process the frequency can be varied.

The reaction temperature of this process is usually elevated, which means that the temperature is more than room temperature. A preferred temperature for the US assisted reaction is above 25°C. More preferred is a range of temperature of 25°C to 150°C.

For the experiments carried out for this patent application commercially available ultrasound laboratory equipment was used from DANACAMERINI s.a.s. di Buffa Cesare & C., Str. del Fioccardo, Turin, Italy. Other useful similar equipment can be bought from Emerson Industrial Automation (Branson Sonifier 250/450).

The reaction time of the ultrasound process can vary. The reaction time is the period of time during which the reaction mixture is treated with Ultrasound. Usually it is a short reaction time, which is usually less than two hours. Preferably the reaction time is between 5 and 60 minutes.

The reaction is usually carried out in a suitable solvent or a mixture of solvents. Suitable solvents are water, alcohols, ethers, esters, ketones, amides, and nitriles. Preferred solvents are water, acetone, ethylene glycol, methanol, ethanol, n-propanol and 2-propanol.

A Pd salt or a mixture of Pd salts are used in the process, especially such salts which are low soluble in water. Suitable Pd(ll) salts are Pd(II)actetate (Pd(OAc)₂). In the prior art usually Pd(ll) chloride salts are used, which cause problems (such as corrosion). Therefore the use of Pd(OAc)₂ is an advantage.

### (2) (Ultrasound assisted) process with cetyldimethyl(2-hydroxyethyl)ammonium dihydrogen phosphate:

Cetyldimethyl(2-hydroxyethyl)ammonium dihydrogen phosphate, which is also known as Luviquat^{™} mono CP AT1 (tradename from BASF) and also as dimethylhexadecyl(2-hydroxyethyl)ammonium dihydrogen phosphate, is used for this process.

To a diluted aqueous solution of cetyldimethyl(2-hydroxyethyl)ammonium dihydrogen phosphate a water soluble Pd(II)-salt (or a mixture of such salts) is added and this reaction mixture is heated for a period of time. Afterwards the so obtained dispersion (or parts of it) is added to an aqueous suspension of carrier material (AIO(OH)). This mixture is stirred and afterwards the pH of this reaction mixture is adjusted to about 10 and then the solid is filtered, washed and dried to obtain the catalytic system.

The reaction is usually carried out in water as solvent.

The concentration of cetyldimethyl(2-hydroxyethyl)ammonium dihydrogen phosphate is usually up to 50 wt-%, based on the weight of water and cetyldimethyl(2-hydroxyethyl)ammonium dihydrogen phosphate. Preferably the concentration of cetyldimethyl(2-hydroxyethyl)ammonium dihydrogen phosphate is 15 - 40 wt-%.

The reaction temperature can vary. The reaction is usually carried out at elevated temperatures. Preferably, the reaction is carried out at a temperature between 30 - 150°C, more preferably at a temperature between 40 - 100°C.

The same process can also be carried out by using a water-insoluble Pd(II)-salt or a mixture of water-insoluble Pd(II)-salts, such as Pd(II)acetate. In this case a sonication step is necessary.

Furthermore is also possible to carry out this process as an one-pot procedure in which both the palladium salt and the carrier material (=AIO(OH)) were submitted to ultrasound altogether in the presence of cetyldimethyl(2-hydroxyethyl)ammonium dihydrogen phosphate in water.

### (3) Ultrasound (US)- and microwave (MW)-assisted process under hydrogen pressure

A Pd(ll) salt (or a mixture of Pd(ll) salts) is suspended in water and then sonicated to get a dispersion that is then reduced under H₂ pressure with MW heating.

The so obtained homogeneous black dispersion (or parts of it) is added to an aqueous suspension of carrier material (AIO(OH)). This mixture is stirred and afterwards the solid is filtered, washed and dried to obtain the catalytic system.

All reaction conditions for the ultrasound assisted process step as already defined under (1) are also to be applied for this ultrasound assisted process step.

After the ultrasound (US)-assisted process step, a microwave (MW)-heating step is carried out.

The length of microwave which is applied during the second reaction step can vary, but usually it is chosen in such a way that it can heat the solvent. Therefore the wavelength is usually from 7.5 cm to 15 cm (frequency 2 GHz to 4 GHz). But the wavelengths could also be larger more than 30 cm. The preferred frequency is 2.45 GHz, approximately 12.2 cm.

Usually a microwave power of at least 100 W is used. Usually the used microwave power lies in the range of 100 W to 2000 W.

For the experiments carried out for this patent application commercially available microwave lab equipment was used (SynthWAVE from MLS GmbH (Germany)).

It also possible to use a process wherein just a MW-step (and no US-step) is carried out.

Selective hydrogenation are such wherein the C-C triple bond is hydrogenates to the corresponding C-C double. The C-C triple bond is not fully hydrogenated. This is a very important, essential and distinguishing property of the process according to the present invention.

The selective hydrogenation can be carried using commonly used reaction conditions in regard to for example H₂ pressure and reaction temperature.

The catalytic systems used in the process according to the present invention are particularly suitable to selectively hydrogenate phenyl acetylene (compound (1)), diphenyl acetylene (compound (2)), buta-2-yn-1,4,-diol (compound (3), 3-hexyne (compound (4)) and compounds of formula (5). 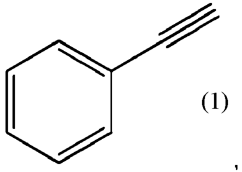 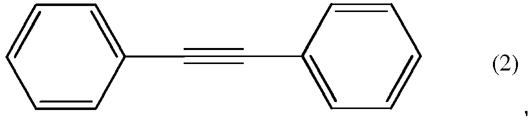 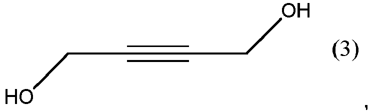 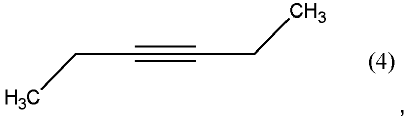 and 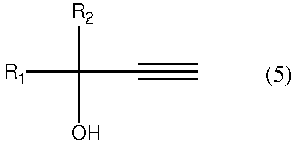
wherein R₁ is C₁ - C₂₀ alkyl or C₃ - C₂₀ alkenyl,
and R₂ is C₁-C₂-alkyl.

Therefore the present invention relates to a selective hydrogenation (SH') of a compound of formula (1), (2), (3), (4) or (5) 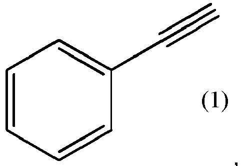 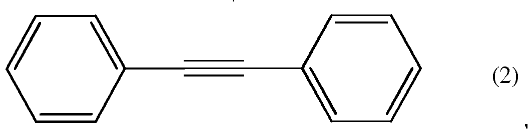 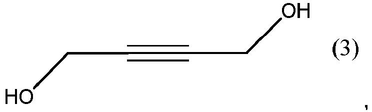 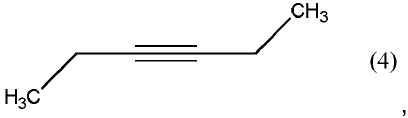 and 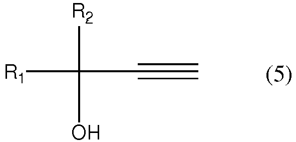
wherein R₁ is C₁ - C₂₀ alkyl or C₃ - C₂₀ alkenyl,
and R₂ is C₁-C₂-alkyl,
characterized in that a catalyst as defined in claim 1 is used.

The corresponding selectively hydrogenated compounds are the following 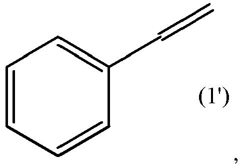 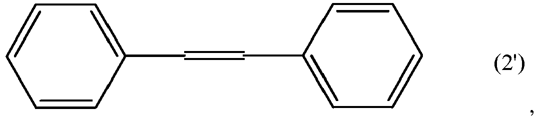 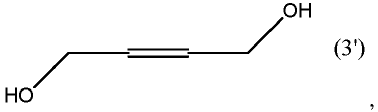 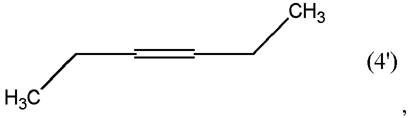 and

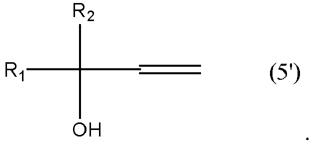

The following compounds of formulae (5a), (5b) and (5c) are preferred compounds of formula (5):

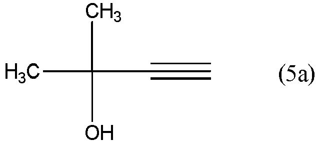

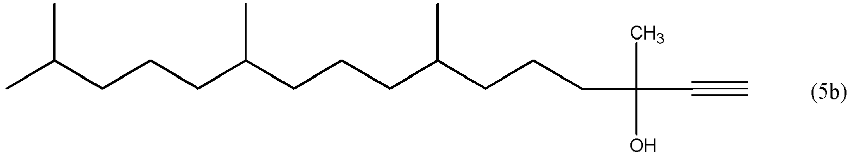

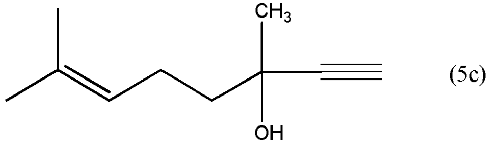

Therefore the present invention relates to a selective hydrogenation (SH") of a compound of formula (5a), (5b) or (5c) 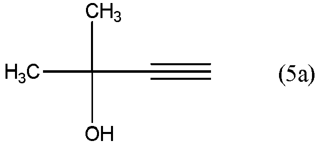 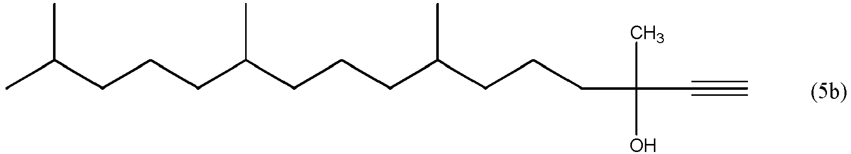 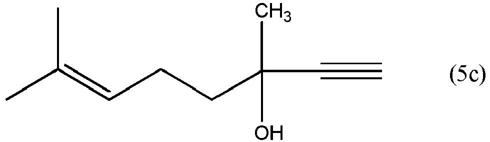 characterized in that a catalyst as defined in claim 1 is used.

The reaction products obtained by the selective hydrogenation are obtained in excellent yield with excellent selectivity.

The following examples serve to illustrate the invention. All percentages and parts (if not otherwise stated) are related to the weight and the temperature is given in °C.

### Examples

### Process of production of the catalysts

### Examples 1a - 1b

Pd(OAc)₂ (20 mg) was suspended in 20 mL of ethylene glycol (orange palladium salt was suspended in the solvent, which remained transparent and colourless) and sonicated with a titanium immersion horn (21.1 kHz at 100 W; 30mm diameter of the horn tip and 170mm length) for 5 min at around 100 °C. After sonication, the suspension became a homogeneous black dispersion that was added dropwise (20 min) to 10 mL of stirring suspension of the support (1 g) with ethylene glycol. The mixture was stirred overnight at room temperature. Then the solid was filtered, washed with methanol and dried under vacuum.

This process was carried out with Boehmite as carrier material. The following table summarized the various catalyst, which have been produced. The weight-% (wt-%) in the table is related to the total weight of the dried catalyst.

| **Exp.** | **Pd-conc.** |
|---|---|
| | **[wt-%], based on the total of catalyst** |
| **1a** | 0.93 |

### Example 2a

500 µL of hexadecyl(2-hydroxyethyl)dimethylammonium dihydrogen phosphate 30% solution in water (Luviquat mono CP AT1) was diluted with distilled water to a final volume of 10 mL.

Water-soluble Na₂PdCl₄ (7.5 mg) was added and the solution (orange solution) was heated in a silicon oil bath for 1 h at 80°C. After heating, the solution became a homogeneous black dispersion that was added dropwise to a stirring suspension of the support (750 mg) in 10 mL of water (within 20 min). The mixture was stirred overnight at room temperature. The pH was adjusted around 10, then the solid was filtered, washed with water and dried under vacuum.

This process was carried out with Boehmite as carrier material. The following table summarized the details of the catalyst, which has been produced. The weight-% (wt-%) in the table is related to the total weight of the dried catalyst.

| **Exp.** | **Pd-conc.** |
|---|---|
| | **[wt-%]** |
| 2a | 0.36 |

### Examples 3a - 3b

The same reaction conditions as in Example 2 were used, but Pd(OAc)₂ (15 mg for example 3a, 7.5 mg for example 3b) was used as Pd(ll) salt, which is water insoluble. Ultrasound (the same device and conditions as in Example 1) was used to disperse the salt in water. Boehmite (750 mg) was the support. Otherwise the reaction was carried out as in Example 2. The weight-% (wt-%) in the table is related to the total weight of the dried catalyst.

| **Exp.** | **Pd-conc.** |
|---|---|
| | **[wt-%]** |
| 3a | 0.93 |
| 3b | 0.47 |

### Examples 4a and 4b

Example 3 was adapted in a one-pot procedure in which both palladium salt and support were sonicated altogether in the presence of Luviquat^{™} mono CP AT1 in water. In other words, sonication of Pd(OAc)₂, H₂O, Luviquat^{™} and support was performed in a cup-horn apparatus (cavitating tube, 19.9 kHz, 100 W; 30mm diameter of the horn tip, 35mm internal diameter and 110mm length of the horn) for 30 min at 30°C. Then the reduction and impregnation was carried out in an oil bath for 2 h at 80°C.

The mixture was stirred overnight at room temperature, and then the solid was filtered, washed with water and dried under vacuum.

This process was carried out with Boehmite as carrier material. The following table summarized the details of the catalysts, which have been produced. The weight-% (wt-%) in the table is related to the total weight of the dried catalyst.

| **Exp.** | **Pd-conc.** |
|---|---|
| | **[wt-%]** |
| 4a | 0.93 |
| 4b | 0.47 |

### Examples 5a - 5c

A Pd(OAc)₂ (15 mg) suspension in water (10 mL) was sonicated in a cup-horn apparatus (cavitating tube, 19.9 kHz, 100 W; 0mm diameter of the horn tip, 35mm internal diameter and 110mm length of the horn) for 30 min at 30°C to get a dispersion that was reduced under 10 bar of H₂ with MW heating (SynthWAVE, Milestone), modulating time, power and temperature.

The homogeneous black dispersion obtained was added dropwise (20 min) to a stirring suspension of support (750 mg) with water (10 mL). The mixture was stirred overnight at room temperature. Then the solid was filtered, washed with water and dried under vacuum.

This process was carried out with Boehmite as carrier material. The following table summarized the various catalyst, which have been produced. (The weight-% (wt-%) in the table is related to the total weight of the dried catalyst.) Furthermore the conditions for the Pd reduction are listed as well (modulating time, power and temperature).

| **Exp.** | **Pd-conc.** | **Reduction of Pd** |
|---|---|---|
| | **[wt-%]** | |
| 5a | 0.93 | 19.9kHz,30min,30°C+MW,12min,80°C |
| 5b | 0.93 | 19.9kHz,30min,30°C+ MW,6min,40°C |
| 5c | 0.93 | 19.9kHz,30min,30°C+MW,6min,40°C+ 19.9kHz,30min,30°C |

### Examples 6a - 6s

### Selective hydrogenation using the catalysts of examples 1 - 5

The supported catalysts obtained by Examples 1 - 5 were tested in conventional hydrogenation reactions of selected alkynes: phenyl acetylene (PA) and diphenylacetylene (DPA). Both PA and DPA were hydrogenated with the prepared Pd-catalysts at room temperature and atmospheric hydrogen pressure. 2 mg of Pd-catalyst was added in a 2 mL of hexane containing 0.112 mmol of alkyne. Each hydrogenation process was performed in duplicate in one multi-tube reactor and analyzed by GC/MS.

In the following table the results of the PA hydrogenation are summarized

| **Exp.** | **cat** | **Temp** | **Time** | **Selectivity** | **Conversion** |
|---|---|---|---|---|---|
| | | **[°C]** | **[min]** | **[%]** | **[%]** |
| **6a** | 1a | 20 | 75 | 97 | 92 |
| **6b** | 1a | 27 | 70 | 93 | 99 |
| **6c** | 2a | 27 | 220 | 96 | 91 |
| **6d** | 3a | 25 | 60 | 96 | 83 |
| **6e** | 3b | 27 | 100 | 97 | 74 |
| **6f** | 4a | 27 | 50 | 95 | 94 |
| **6g** | 4b | 27 | 100 | 91 | 99 |
| **6h** | 5b | 27 | 50 | 92 | 100 |

In the following table the results of the DPA hydrogenation are summarized

| **Exp.** | **cat** | **Temp** | **Time** | **Selectivity** | **Conversion** |
|---|---|---|---|---|---|
| | | **[°C]** | **[min]** | **[%]** | **[%]** |
| **6i** | 2a | 27 | 110 | 86 | 97 |
| **6j** | 3a | 25 | 30 | 84 | 94 |
| **6k** | 4a | 25 | 30 | 86 | 96 |
| **6l** | 5a | 27 | 68 | 84 | 97 |
| **6m** | 5c | 27 | 68 | 82 | 98 |

The selectivity and the conversion of the selective hydrogenation using the catalytic systems according to the present invention are excellent.

### Hydrogenation of Methylbutynol to Methylbutenol

A solution of 50 g methylbutynol in 200 g heptane and 100 mg of the catalyst (see following table) was added to a 500ml autoclave. The reaction mixture was purged 3 times with nitrogen (pressurise to 6 bar absolute and release). Then the mixture was heated to 60 °C and purged 3 times with hydrogen (pressurise to 6 bar absolute and release). The mixture was pressurised to 3 bar hydrogen (absolute) and stirred at 2000 rpm. When the desired amount of hydrogen had been consumed, samples were taken and the reaction mixture was cooled to room temperature.

| **Exp.** | **cat** | **Temp** | **Time** | **Selectivity** | **Conversion** |
|---|---|---|---|---|---|
| | | **[°C]** | **[min]** | **[%]** | **[%]** |
| **7a** | 4a | 60 | 107 | 95 | >99 |
| **7b** | 5b | 60 | 234 | 96 | 99 |

## Claims

1. Selective hydrogenation of C=C triple bonds to C=C double bonds **characterized in that** at least one catalytic system (I) comprising
(a) Pd-nanoparticles on
(b) AlO(OH) as carrier material
is used.

2. Selective hydrogenation according to claim 1 **characterized in that** the catalytic system does not contain any lead.

3. Selective hydrogenation according to any of the preceding claims wherein up to 8 weight-% (wt-%), based on the total weight of the catalytic system, of Pd is on the carrier material.

4. Selective hydrogenation according to any of the preceding claims wherein at least 0.01 wt-%, based on the total weight of the catalytic system, of Pd is on the carrier material.

5. Selective hydrogenation according to any of the preceding claims, wherein 0.01 - 5 wt-%, based on the total weight of the catalytic system, of Pd is on the carrier material.

6. Selective hydrogenation according to claim 1, **characterized in that** one of the following compound is hydrogenated selectively 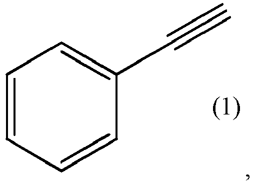 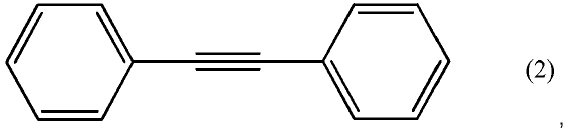 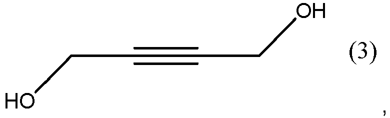 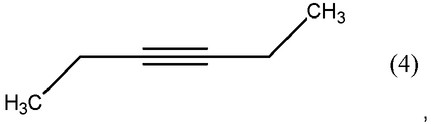 and 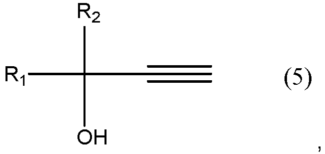
wherein R₁ is C₁ - C₂₀ alkyl or C₃ - C₂₀ alkenyl,
and R₂ is C₁-C₂-alkyl.

## Patentansprüche

1. Selektive Hydrierung von C≡C-Dreifachbindungen zu C=C-Doppelbindungen, **dadurch gekennzeichnet, dass** mindestens ein katalytisches System (1), umfassend
(a) Pd-Nanoteilchen auf
(b) A10(OH) als Trägermaterial,
verwendet wird.

2. Selektive Hydrierung nach Anspruch 1, **dadurch gekennzeichnet, dass** das katalytische System kein Blei enthält.

3. Selektive Hydrierung nach einem der vorhergehenden Ansprüche, wobei bis zu 8 Gewichts-% (Gew.-%), bezogen auf das Gesamtgewicht des katalytischen Systems, Pd auf dem Trägermaterial vorliegen.

4. Selektive Hydrierung nach einem der vorhergehenden Ansprüche, wobei mindestens 0,01 Gew.-%, bezogen auf das Gesamtgewicht des katalytischen Systems, Pd auf dem Trägermaterial vorliegen.

5. Selektive Hydrierung nach einem der vorhergehenden Ansprüche, wobei 0,01-5 Gew.-%, bezogen auf das Gesamtgewicht des katalytischen Systems, Pd auf dem Trägermaterial vorliegen.

6. Selektive Hydrierung nach Anspruch 1, **dadurch gekennzeichnet, dass** eine der folgenden Verbindungen selektiv hydriert wird: 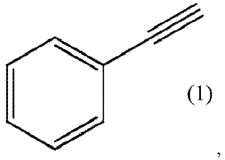 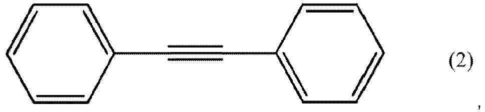 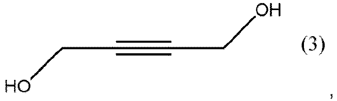 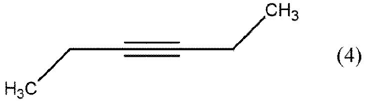 und 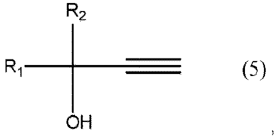
wobei R₁ für C₁-C₂₀-Alkyl oder C₃-C₂₀-Alkenyl steht
und R₂ für C₁-C₂-Alkyl steht.

## Revendications

1. Hydrogénation sélective de triples liaisons C≡C en doubles liaisons C=C **caractérisée en ce qu'**au moins un système catalytique (1) comprenant
(a) des nanoparticules de Pd sur
(b) AlO(OH) comme matériau de support
est utilisé.

2. Hydrogénation sélective selon la revendication 1 **caractérisée en ce que** le système catalytique ne contient pas de plomb.

3. Hydrogénation sélective selon l'une quelconque des revendications précédentes, jusqu'à 8 % en poids, sur la base du poids total du système catalytique, de Pd étant sur le matériau de support.

4. Hydrogénation sélective selon l'une quelconque des revendications précédentes, au moins 0,01 % en poids, sur la base du poids total du système catalytique, de Pd étant sur le matériau de support.

5. Hydrogénation sélective selon l'une quelconque des revendications précédentes, 0,01 à 5 % en poids, sur la base du poids total du système catalytique, de Pd étant sur le matériau de support.

6. Hydrogénation sélective selon la revendication 1, **caractérisée en ce que** l'un des composés suivants est hydrogéné sélectivement 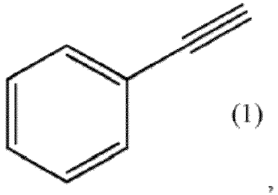 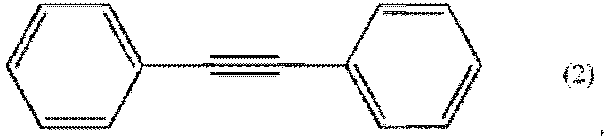 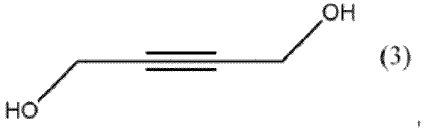 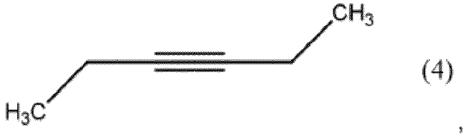 et 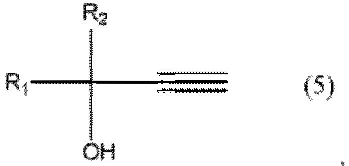
R₁ étant alkyle en C₁₋₂₀ ou alcényle en C₃₋₂₀,
et R₂ étant alkyle en C₁₋₂ .
